# EUROPEAN PATENT APPLICATION

(11) **EP 2 500 112 A2**
(43) Date of publication of application: **19.09.2012**
(21) Application number: 12460008.1
(22) Date of filing: 28.02.2012
(51) Int. Cl.: B09B 3/00

(54) **A micronizer for crop wastes, animal farming and slaughter wastes and for other biodegradable materials including municipal wastes**

(30) Priority: 09.03.2011 PL 39416811
(71) Applicant: PL-USA Centrum Badawczo Wdrozeniowe Nowych Technologii Spolka z ograniczona odpowiedzianoscia, 59-800 Luban (PL)
(72) Inventor: Mazurkiewicz, Marian, 52-205 Wroclaw (PL); Pleszkun, Andrzej, 59-620 Gryfow Slaski (PL); Czyrko, Angelika, 59-800 Luban (PL)
(74) Representative: Kozlowska, Regina

(57) **Abstract**

The object of the invention is a plant for micronization of crop wastes, animal farming and slaughter wastes and other biodegradable materials including municipal wastes, designed for the preparation of a feedstock for biogas generation plants.

Said plant comprises the first screw feeder (4) driven by the first motor (5) and placed at the bottom (3) of the working chamber (1), said chamber is in form of a truncated cone and terminates in a choke (6) shaped as a perforated truncated cone. The outlet of the choke (6) of the working chamber (1) is placed in a product chamber (13), wherein mounted are rotary heads (9) generating high-energy fluid jets (10), pointed towards the outlet of the choke (6). The product chamber (13) is provided with the second screw feeder (15) driven by the second motor (16) and mounted above a chute (14) used for guiding the generated biogas material into a product tank (17) provided with a mixer (20) and temperature sensor (21). From the product tank (17) the biogas material is transported to biogas material tanks (19) by a pipe provided with the first pump (18), and/or to a tank-truck (23) by pipes provided with the second pump (22).

## Description

The present invention relates to a micronizer for crop wastes, animal farming and slaughter wastes and other biodegradable materials including municipal wastes, designed for preparation of a feedstock for biogas generation plants. The feedstock for biogas generation plants is prepared on machines which are not efficient enough to guarantee the requested fineness of the prepared wastes. Such agricultural wastes as straw, grass, beet leaves, plant stalks, fruit peduncles, fruit waste, also the dung- from farm animals, animal slaughter waste etc. are subjected to a milling or grinding process. The time of the biogas production process and its efficiency in the first place depend on the specific surface of feedstock particles. In order to increase the efficiency of the biogas production process, particles in the feedstock should be broken up as finely as possible, so as to make significantly easier access for waste-digestive microorganisms.

A method and a plant for micronization and separation of municipal solid wastes by high-pressure fluid jets has been known from a Polish patent specification No. PL199667, wherein said jets, while colliding with soft organic fractions, are capable of shooting through, cutting, breaking up and separating materials to a degree depending on mechanical properties thereof. The method consists in that the municipal solid wastes are transported to a working space and, while moving through it, the wastes are subjected to high-energy fluid jets generated by a set of nozzles supplied by a high pressure pump, which leads as a result to separation of soft fractions and organic solid wastes from insoluble solid fractions. Washed out insoluble solid fractions are disposed to a tank for solid particles, whereas disintegrated and/or dissolved and washed out soft fractions and organic wastes in the form of a suspension are removed to a tank for washed out organic particles. The suspension is homogenized and collected in a sedimentation tank, and the fluid decanted therefrom is after a treatment returned to the set of nozzles. Furthermore, in the set of nozzles generated are high-power, steady and/or rotating fluid jets, pressurized to 20 to 3000 atm. The working space in the plant is provided with fittings for loading solid wastes, and with a set of nozzles connected to a high pressure feeding pipe supplied by a high pressure pump. The working space is further provided with perforated components, especially sieves to carry away the particles that have been washed out by high-energy jets, to a tank for organic wastes. The working space is connected to a tank for separated solid particles. The working space forms a kind of a cylindrical tank, with its axis inclined towards a flat base. Inside this cylindrical tank there is a conveyor screw driven by a motor through a gearbox, and under the conveyor screw a sieve is placed for separation of washed out organic particles, which are then transferred to a storage tank for washed out organic particles, from solid particles, which are then transferred to a storage tank for solid particles. Both storage tanks are located under the said cylindrical tank. Furthermore, the plant is provided with a feed hopper for feeding solid wastes into the cylindrical tank, straight under the set of nozzles that are put in rotation by nozzle motors. The storage tank for washed out organic particles is located under the cylindrical tank, from the feed hopper side, whereas the storage tank for solid particles is located on the opposite side of the hopper. The nozzles are swivel-mounted in the high-pressure feeding pipe with the aid of nozzle bodies. The nozzles are put in rotation by nozzle motors, and the nozzle exhaust diameter ranges from 0.1 mm to 5 mm. A belt feeder for carrying solid wastes is located in front of the charging hopper, and a magnetic separator for removal of ferrous fractions is mounted above the belt feeder.

A method of producing a microsuspension and a micronizing plant for production of the same have been known from a Polish patent application No. P.385162, and used for hydroseparation of municipal waste by high-energy fluid jets, which hit the free-passing garbage stock, comminute organic components, separate and wash them out of the garbage stock. The method consists in that a high pressure pump, supplying a primary nozzle, generates an ultra-high-speed jet of process fluid, said jet is used in a micronizing compartment supplied with material being comminuted, to produce a high-speed jet of solid particles suspended in the process fluid, which is then shot through a secondary nozzle into a pipe enclosure of a homogenizing chamber, wherein the comminuted material is further micronized and homogenized, and the finished product is collected in a tank. Such a micronizer is a high-power plant, provided with a high-pressure pump supplying a primary nozzle, generating a high-speed fluid jet, and mounted inside a micronizing chamber that is fed from a feeder with the material awaiting the comminution. A secondary nozzle is located in the micronizing chamber on the opposite side of the primary nozzle and aligned with the primary nozzle. A homogenizing chamber, thereinto the suspension jet is shot, is located downstream from the enclosure of the micronizing chamber, and terminates in a stub pipe.

The essence of the plant according to the present invention consists in that a first screw feeder driven by a first motor is placed at the bottom of a working chamber, said chamber is in form of a truncated cone and terminates in a choke shaped as a perforated truncated cone. The choke outlet of a working chamber is placed in a product chamber wherein mounted are rotary heads that generate high-energy fluid jets pointed towards the choke outlet. A second screw feeder driven by a second motor is placed in a product chamber, above a chute for guiding the generated biogas material into a product tank provided with a mixer and temperature sensor, wherefrom the biogas material is transported to biogas material tanks by a pipe provided with a first pump and/or to a tank-truck - by pipes provided with a second pump.

Preferably, rotary heads generating high-energy fluid jets towards feed material transported by the first screw feeder from the working chamber to the choke outlet, are mounted on a side surface of the choke cone.

Preferably, the choke perforation is in the form of holes.

It is advantageous if the angle α of the choke cone and the diameter of the choke outlet are selected with regard to properties of the feed material as well as the assumed output of the plant, where the angle α of the choke cone ranges from 5 to 30 degrees, and the choke diameter ranges from 10 cm to 100 cm.

Preferably, the rotary heads generating high-energy fluid jets are pointed frontally towards the choke outlet, and their speed is controlled within the range from 0 to 25000 rpm.

Preferably, the number of rotary heads and the number of fluid jets are selected with regard to the planned output and desired degree of the feed material disintegration.

Preferably, the pressure generated by the high-pressure pump is selected with regard to properties of the feed material as well as the assumed output of said plant and the desired degree of the feed material disintegration.

Preferably, the feed material consists of agricultural wastes and/or animal slaughter wastes and/or organic matter.

A plant according to the invention, provided with high-energy jets, makes it possible to disintegrate the feed material to an average particle size far below 50 micrometers, owing to which the biogas material fermentation process needs significantly shorter time, while the specific surface of the material substantially increases. Efficiency of the biogas production depends on the specific surface of particles of the organic matter being fermented.

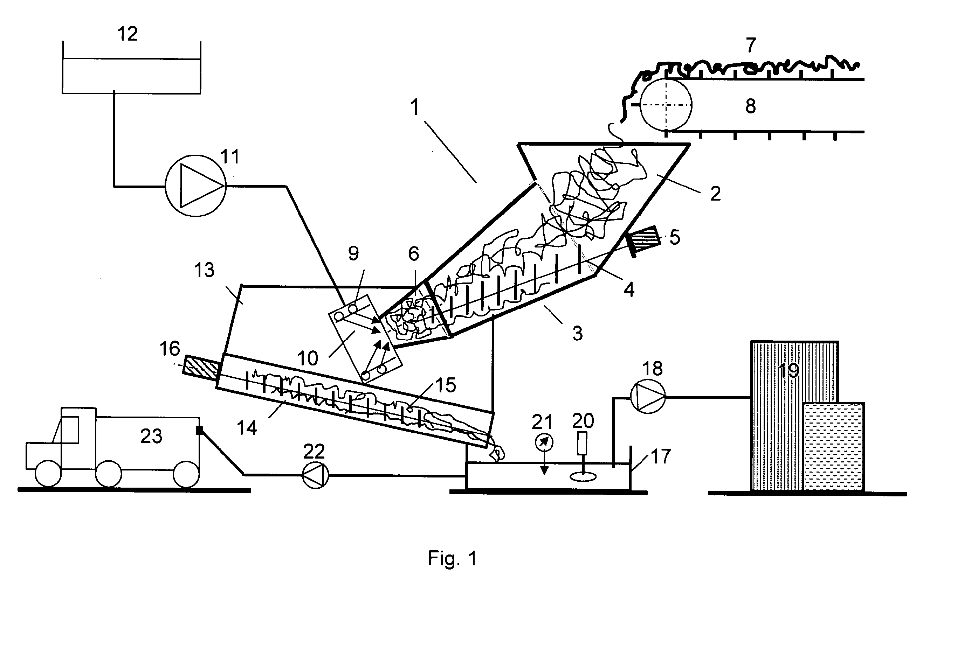

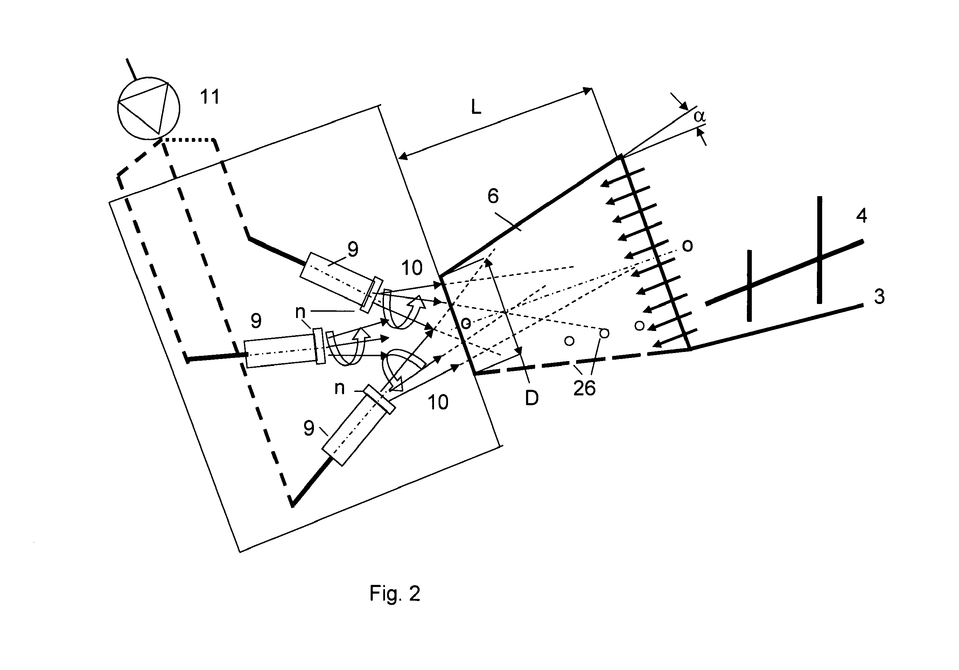

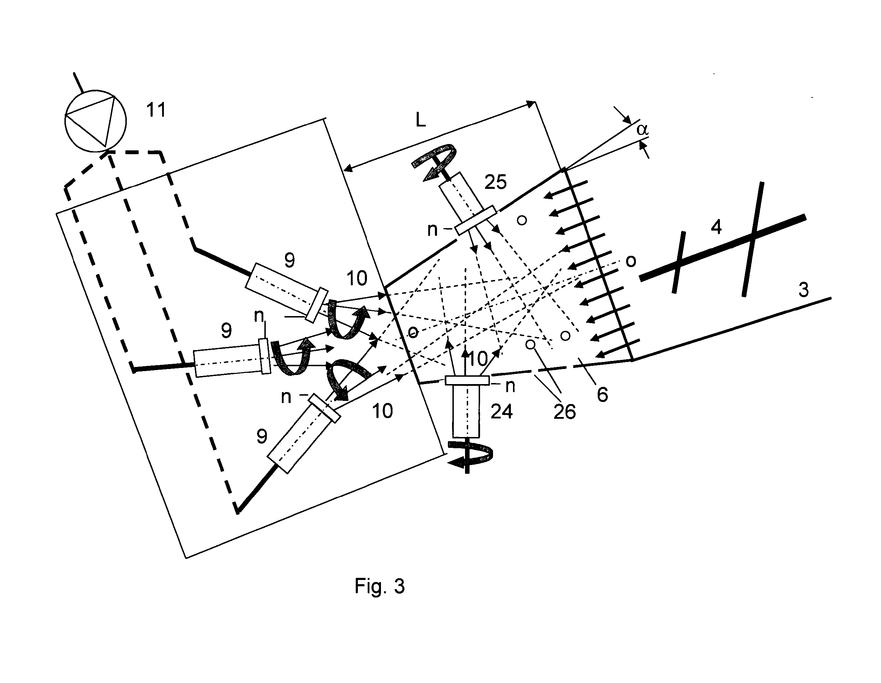

The object of the invention in an embodiment has been shown in drawings, in which: fig. 1 shows a plant for micronization of wastes, fig. 2 - shows a choke provided with a set of rotary heads which are located from the choke outlet side, and fig. 3 - shows a choke provided with a set of rotary heads which are located from the choke outlet side and on side surface of the choke cone.

A plant for the micronization of crop wastes, animal farming and slaughter wastes and other biodegradable materials including municipal wastes, comprises a working chamber 1 provided with a first screw feeder 4 driven by a first motor 5, whereinto feed material 7 is transported through a loading chamber 2 by means of a conveyor 8. The working chamber 1 is in form of a truncated cone and terminates in a choke 6, shaped as a perforated truncated cone. The first screw feeder 4 is placed at the bottom 3 of the working chamber. The outlet of the choke 6 of the working chamber 1 is placed in a product chamber 13, wherein mounted are rotary heads 9 generating high-energy fluid jets 10 and pointed towards the outlet of the choke 6. Perforation of the choke 6 is in the form of holes 26. Fluid supplied to rotary heads 9 is pumped from a fluid tank 12 by a high-pressure pump 11. The rotary heads 9 generating high-energy fluid jets 10 are pointed frontally towards the outlet of the choke 6, and their speed is controlled within the range from 0 to 2500 rpm. The product chamber 13 is located under the working chamber 1, and is provided with a second screw feeder 15 driven by a second motor 16, said screw feeder 15 is mounted above a chute 14 designed for guiding the generated biogas material into a product tank 17 provided with a mixer 20 and temperature sensor 21. From the product tank 17 the biogas material is transported to biogas material tanks 19 by a pipe provided with a first pump 18 and to a tank-truck 23 by pipes provided with a second pump 22.

The plant for micronization of wastes can be further provided with rotary heads 24, 25 mounted on side surface of the choke 6 cone, said heads generate high-energy fluid jets 10, pointed towards the feed material 7 which is transported by the first screw feeder 4 from the working chamber 1 to the outlet of the choke 6. The rotary heads 24 and 25 mounted on side surface of the choke 6, intensify the process of disintegration of the feed material 7.

The number of rotary heads 9, 24, 25, the number of fluid jets 10, the pressure generated by the high-pressure pump 11, the cone angle α of the choke 6 and the diameter of the choke 6 are selected with regard to the planned output of the plant, the properties of the feed material 7 and the desired disintegration degree of the same, while the cone angle α of the choke 6 ranges from 5 to 30 degrees, and the outlet diameter of the choke 6 ranges from 10 cm to 100 cm; the size of the angle α, which defines conic convergence of the choke 6 and the length L of the choke cone, are crucial factors for the degree of the material compaction in the choke 6. Furthermore, the rotary heads 9, 24, 25 can be provided with nozzles of mouth diameters ranging from 0.1mm to 5 mm. The feed material 7 consists of agricultural wastes, animal slaughter wastes, organic matter and all kinds of biodegradable materials, including municipal wastes. The process of disintegration depends on the number of applied rotary heads 9, 24, 25, their speed and the number of generated fluid jets 10. Another parameter of the disintegration process is also the distance between rotary heads 9 and the choke 6.

The loose feed material 7 is put in the plant according to the invention to continuous and forced micronization, said feed material is transported by the first screw feeder 4 to the working chamber 1, wherein it undergoes compaction, and as a dense stock it is transported to the choke 6. The high-energy fluid jets 10, flowing under high pressure from the rotary heads 9 that are mounted at the outlet of the choke 6, disintegrate the inflowing material at the rate comparable to the rate of sound propagation in the air. Nearly 100% of the energy of the jets is utilised as their lines of impact constantly inscribe into the stock of the inflowing feed material 7. Rotation speed of the heads 9, their number, number of fluid jets 10 generated in each head, and the distance between the nozzles and the outlet of the choke 6 are factors that decide on the degree of the feed material disintegration at the assumed quantity thereof. Ratio by weight of disintegrated quantity of the feed material 7 to quantity of the fluid consumed in the disintegration process depends on the quantity of the feed material 7, the total cross-sectional area of the used nozzles and the applied fluid pressure. Effectiveness of disintegration by fluid jets 10 is conditioned by the degree of the material compaction at the outlet of the choke 6, therefore the more concentrated are the lines of fluid jets 10 in the plane of contact with the feed material, the smaller particles of the disintegrated feed material, and thus the larger specific surface of the product which is the biogas material, can be expected.
**List of designations used in the drawing:**
- 1.: Working chamber,
- 2.: Loading chamber,
- 3.: Bottom of chamber,
- 4.: First screw feeder,
- 5.: First motor,
- 6.: Choke of disintegration chamber,
- 7.: Material,
- 8.: Conveyor,
- 9.: Set of rotary heads,
- 10.: High-energy fluid jets,
- 11.: High-pressure pump,
- 12.: Fluid tank,
- 13.: Product chamber,
- 14.: Chute,
- 15.: Second screw feeder,
- 16.: Second motor,
- 17.: Product tank,
- 18.: First pump,
- 19.: Biogas material tanks,
- 20.: Mixer,
- 21.: Temperature sensor,
- 22.: Second pump,
- 23.: Tank-truck,
- 24.: Rotary head,
- 25.: Rotary head,
- 26.: Hole.

## Claims

1. A plant for micronization of crop wastes, animal farming and slaughter wastes and other biodegradable materials including municipal wastes, comprising a working chamber provided with a screw feeder driven by a motor, thereinto a feed material is transported through a loading chamber by means of a conveyor, and a product chamber located under the working chamber, furthermore comprising rotary heads are provided with nozzles and supplied by a high-pressure pump, while the nozzle exhaust diameters range from 0.1 mm to 5 mm, wherein the first screw feeder (4) driven by the first motor (5) is placed at the bottom (3) of the working chamber (1), said chamber is in the form of a truncated cone and terminates in a choke (6) shaped as a perforated truncated cone, whereas the outlet of the choke (6) of the working chamber (1) is placed in a product chamber (13), where mounted are rotary heads (9) generating high-energy fluid jets (10), pointed towards the outlet of the choke (6), said product chamber (13) is further provided with the second screw feeder (15) driven by the second motor (16) and mounted above a chute (14) used for guiding the generated biogas material into a product tank (17) provided with a mixer (20) and temperature sensor (21), and from said product tank (17) the biogas material is transported to biogas material tanks (19) by a pipe provided with the first pump (18), and/or to a tank-truck (23) by pipes provided with the second pump (22).

2. A plant according to claim 1, wherein rotary heads (24, 25) generating high-energy fluid jets (10) towards the feed material (7) transported by the first screw feeder (4) from the working chamber (1) to the outlet of the choke (6) are mounted on a side surface of the cone of the choke (6).

3. A plant according to claim 1, wherein perforation of the choke (6) is made in the form of holes (26).

4. A plant according to claim 1, wherein the cone angle α of the choke (6) and the diameter of the choke (6) are selected with regard to properties of the feed material as well as the assumed output of said plant, while said cone angle α of the choke (6) ranges from 5 to 30 degrees, and the diameter of said choke (6) outlet ranges from 10 cm to 100 cm.

5. A plant according to claim 1, wherein said rotary heads (9) generating high-energy fluid jets (10) are pointed frontally towards the outlet of said choke (6), and their speed is controlled within the range from 0 to 25000 rpm.

6. A plant according to claim 1 and 2, wherein the number of said rotary heads (9, 24, 25) and the number of said fluid jets (10) are selected with regard to the planned output and desired degree of the feed material (7) disintegration.

7. A plant according to claim 1, wherein the pressure generated by said high-pressure pump (11) is selected with regard to properties of said feed material (7) as well as the planned output and desired degree of said feed material (7) disintegration.

8. A plant according to claim 1, wherein said feed material (7) consists of agricultural wastes.

9. A plant according to claim 1, wherein said feed material (7) consists of animal slaughter wastes.

10. A plant according to claim 1, wherein said feed material (7) consists of organic matter.
